# EUROPEAN PATENT SPECIFICATION

(11) **EP 0 522 494 B1**
(45) Date of publication and mention of the grant of the patent: **02.10.1996**
(21) Application number: 92111478.1
(22) Date of filing: 07.07.1992
(51) Int. Cl.: C07D 209/60, A61K 31/40

(54) **Novel isatineoxime derivatives, their preparation and use**
Neue Isatineoxime Derivate, ihre Herstellung und Verwendung
Nouveaux dérivés d'isatinoxime, leur préparation et leur utilisation

(30) Priority: 09.07.1991 US 727479
(43) Date of publication of application: 13.01.1993
(73) Proprietor: NEUROSEARCH A/S, DK-2600 Glostrup (DK)
(72) Inventor: Wätjen, Frank, DK-2730 Herlev (DK); Drejer, Jorgen, DK-3500 Vaerlose (DK); Jensen, Leif Helth, DK-1573 Copenhagen v. (DK)
(74) Representative: Grünecker, Kinkeldey, Stockmair & Schwanhäusser Anwaltssozietät

(56) References cited:
- EP-A- 0 432 648

## Description

The present invention relates to novel ring fused indole-2,3-dione oxime derivatives, a method of treatment therewith, pharmaceutical compositions comprising the compounds and to a method of preparing the novel compounds of the invention.

### Object of the Invention

It is an object of the present invention to provide novel isatine compounds which are useful in the treatment of diseases in mammals, including a human, and especially in the treatment of diseases which can be treated by antagonizing an excitatory amino acid of such mammal.

Another object of the present invention is to provide a method of treating diseases in mammals, including a human, responsive to the blockade of glutamic and aspartic acid receptors which comprises administering to a mammal in need thereof a compound of the invention.

A third object of the present invention is to provide novel pharmaceutical compositions for the treatment of diseases in mammals, including a human, responsive to the blockade of glutamic and aspartic acid receptors.

### Background of the Invention

Excessive excitation by neurotransmitters can cause the degeneration and death of neurons. It is believed that this degeneration is in part mediated by the excitotoxic actions of the excitatory amino acids (EAA) glutamate and aspartate at the N-methyl-D-aspartate (NMDA), the α-amino-3-hydroxy-5-methyl-4-isoxazole propionic acid (AMPA) receptor, and the kainate receptor. This excitotoxic action is responsible for the loss os neurons in cerebrovascular disorders such as cerebral ischemia or cerebral infarction resulting from a range of conditions, such as thromboembolic or haemorrhagic stroke, cerebral vasospasm, hypoglycaemia, cardiac arrest, status epilepticus, perinatal asphyxia, anoxia such as from drowning, pulmonary surgery and cerebral trauma as well as lathyrism, Alzheimer's, Parkinsonism, and Huntington's diseases.

The compounds of the present invention may also be useful in the treatment of schizophrenia, epilepsy, anxiety, pain and drug addiction.

EPA 432648, published June 19, 1991, discloses certain isatineoxime derivatives. Some of the compounds of the present invention are comprised by the generic disclosure of that European Patent Application. However, no compounds having the very important features and substituents as the compounds of the present invention are specifically disclosed in EPA 432648 although some of the compounds of the present invention are disclosed in a broad and generic way therein.

### Summary of the Invention

The invention then, inter alia, comprises the following, alone or in combination:

A compound having the formula 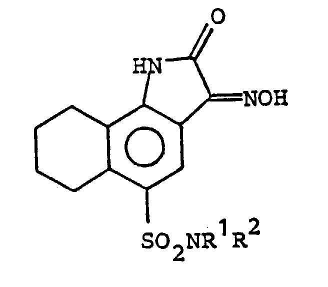 wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein n is 3, 4 or 5,
and a compound as above which is
5-N,N-dimethylsulfamoyl-1H-6,7,8,9-tetrahydrobenz[g]indole-2,3-dione-3-oxime,
and a compound as above which is
5-(1-pyrrolidinylsulfonyl)-1H-6,7,8,9-tetrahydrobenz[g]-indole-2,3-dione-3-oxime,
and a method of treating disorders of a mammal, including a human, responsive to the blockade of glutamic and aspartic acid receptors, which comprises administering to a patient in need thereof a compound as first above in unit dosage form,
and a method as above wherein cerebrovascular disorders, Parkinsonism, anoxia, schizophrenia, migraine, epilepsy, pain, drug addiction, Alzheimer's disease and Huntington's disease are treated,
and further a pharmaceutical composition comprising a therapeutically-effective amount of a compound as first above together with a pharmaceutically-acceptable carrier,
and the use of a compound as first above for the preparation of a medicament useful in the treatment of disorders of a mammal, including a human, responsive to the blockade of glutamic or aspartic acid receptors,
and the use of a compound as first above for the preparation of a medicament useful in the treatment of cerebrovascular disorders, Parkinsonism, anoxia, anxiety, schizophrenia, migraine, epilepsy, pain, drug addiction, Alzheimer's disease and Huntington's disease,
and the use as above wherein the compound is 5-N,N-dimethylsulfamoyl-1H-6,7,8,9-tetrahydrobenz[g]indole-2,3-dione-3-oxime.

### Biological Activity

The compounds of the invention exhibit valuable biological properties because of their strong excitatory amino acid (EAA) antagonizing properties at the AMPA ((RS)-α-amino-3-hydroxy-5-methyl-4-isoxazolepropionic acid) binding site.

Further as compared to known glutamate antagonists acting at the same binding site (see for example EPA 283959) the compounds of the invention are extremely superior because of their high bioavailability.

The compound 5-N,N-dimethylsulfamoyl-1H-6,7,8,9-tetrahydrobenz[g]indole-2,3-dione-3-oxime for example exhibit an IC₅₀ of 2 µM in the AMPA binding assay as described by T. Honoré et al., Neuroscience Letters 54, 27-32 (1985).

The compound 5-N,N-dimethylsulfamoyl-1H-6,7,8,9-tetrahydrobenz[g]indole-2,3-dione-3-oxime has an ED₅₀ of 3 mg/kg when administered i.v. and of 30 mg/kg when administered orally in the AMPA seizure test as described below.

### AMPA-induced clonic seizures

AMPA given icv (intracerebroventricular) (15 µg/kg) NMRI to mice induces clonic seizures which should be inhibited by non-NMDA receptor antagonists.

### Method

Test compound was given i.v. 5 min (or p.o. 30 min) before a 0.3 µg icv administration of AMPA to 10 female NMRI mice (weighing 24-26 g) per dose. The number of mice experiencing clonic seizures within the next 5 min was noted. An ED₅₀ value was calculated as the dose inhibiting 50% of the mice from having clonic seizures.

### Pharmaceutical Compositions

The compounds of the invention, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing ten (10) milligrams of active ingredients or, more broadly, 0.1 to one hundred (100) milligrams, per tablet, are accordingly suitable representative unit dosage forms.

Solid forms of pharmaceutical compositions for p.o. administration and injectable solutions are preferred.

### Method of Treating

The compounds of this invention are extremely useful in the treatment of central nervous system disorders related to their biological activity. The compounds of this invention may accordingly be administered to a subject, including a human, in need of treatment, alleviation, or elimination of an indication associated with the biological activity of the compounds. This includes especially excitatory amino acid dependent psychosis, excitatory amino acid dependent anoxia, excitatory amino acid dependent ischemia, excitatory amino acid dependent convulsions and excitatory amino acid dependent migraine. Suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

### Example 1

### a) 5-chlorosulfonyl-1H-6,7,8,9-tetrahydrobenz[g]indole-2,3-dione

4 g (19.90 mmol) of 1H-6,7,8,9-tetrahydrobenz[g]indole-2,3-dione (prepared as in EPA-432648) is added portionwise to 15 ml chlorosulphonic acid while the temperature is kept at room temperature. The mixture is stirred for 30 minutes and is thereafter poured unto ice water. The precipitate is isolated and dried and is thereafter washed with diethylether/light petroleum and the precipitate is isolated.

### b) 5-N,N-dimethylsulfamoyl-1H-6,7,8,9-tetrabydrobenz[g]-indole-2,3-dione

1 g (3.34 mmol) 5-chlorosulfonyl-1H-6,7,8,9-tetrahydrobenz[g]indole-2,3-dione is suspended in tetrahydrofuran (THF) (25 ml). The mixture is cooled on an ice bath and is exposed to gaseous dimethylamine for 5 minutes. The reaction mixture is evaporated and the residue is washed with ethylacetate/water. The precipitate is isolated by filtration and washed with THF. The crude product is purified by treatment with methylene chloride and ethylacetate. The resulting filtered solution is concentrated in vacuo and the precipitate is isolated and is washed with ethylacetate. Yield 0.24 g, M.p. 162-165°C.

In exactly the same manner the following compound are prepared from pyrrolidine, piperidine, diethylamine and di-(n-butyl)amine.

5-(1-pyrrolidinyl-sulfonyl)-1H-6,7,8,9-tetrahydrobenz-[g]indole-2,3-dione (isolated as an oil),

5-N,N-diethylsulfamoyl-1H-6,7,8,9-tetrahydrobenz[g]-indole-2,3-dione (isolated as an oil),

5-(N,N-di-(n-butyl)sulfamoyl-1H-6,7,8,9-tetrahydrobenz-[g]indole-2,3-dione (isolated as an oil),

5-(1-piperidyl-sulfonyl)-1H-6,7,8,9-tetrahydrobenz[g]-indole-2,3-dione (isolated as an oil).

### c) 5-N,N-dimethylsulfamoyl-1H-6,7,8,9-tetrahydrobenz[g]-indole-2,3-dione-3-oxime

0.2 g (0.65 mmol) of 5-N,N-dimethylsulfamoyl-1H-6,7,8,9-tetrahydrobenz[g]indole-2,3-dione is mixed with 0.05 g (0.71 mmol) hydroxylamine hydrochloride, 0.07 g (0.71 mmol) disodium carbonate and 7 ml of methanol and the mixture is stirred for 2 hours at room temperature. 5 ml water and acetic acid (few drops) is added and the resulting precipitate is isolated. Yield 0.16 g, M.p. 268-270°C.

In exactly the same manner the following compound are prepared:

5-(1-pyrrolidinyl-sulfonyl)-1H-6,7,8,9-tetrahydrobenz[g]indole-2,3-dione-3-oxime, M.p. Brown oil,

5-(1-piperidyl-sulfonyl-1H-6,7,8,9-tetrahydrobenz[g]-indole-2,3-dione-3-oxime, M.p. 256-259°C,

5-N,N-di-(n-butyl)sulfamoyl-1H-6,7,8,9-tetrahydrobenz[g]indole-2,3-dione-3-oxime, M.p. 238-240°C,

5-N,N-diethylsulfamoyl-1H-6,7,8,9-tetrahydrobenz[g]-indole-2,3-dione-3-oxime, M.p. 244-245°C.

## Claims (Claims for the following Contracting State(s): AT, BE, CH, DE, DK, FR, GB, IT, LI, LU, MC, NL, PT, SE)

1. A compound having the formula 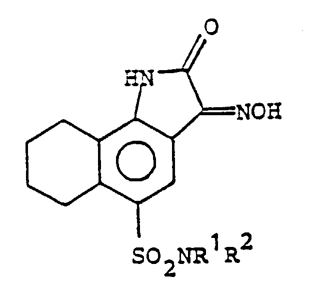 wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein n is 3, 4 or 5.

2. A compound of claim 1 which is
5-N,N-dimethylsulfamoyl-1H-6,7,8,9-tetrahydrobenz[g]-indole-2,3-dione-3-oxime.

3. A compound of claim 1 which is
5-(1-pyrrolidinylsulfonyl)-1H-6,7,8,9-tetrahydrobenz[g]-indole-2,3-dione-3-oxime.

4. A pharmaceutical composition comprising a therapeutically-effective amount of a compound of claim 1 together with a pharmaceutically-acceptable carrier.

5. The use of a compound of claim 1, for the preparation of a medicament useful in the treatment of disorders of a mammal, including a human, responsive to the blockade of glutamic or aspartic acid receptors.

6. The use of a compound of claim 1 for the preparation of a medicament useful in the treatment of cerebrovascular disorders, Parkinsonism, anoxia, anxiety, schizophrenia, migraine, epilepsy, pain, drug addiction, Alzheimer's disease and Huntington's disease.

7. The use of claim 5 wherein the compound is 5-N,N-dimethylsulfamoyl-1H-6,7,8,9-tetrahydrobenz[g]indole-2,3-dione-3-oxime.

8. A method of preparing a compound having the formula 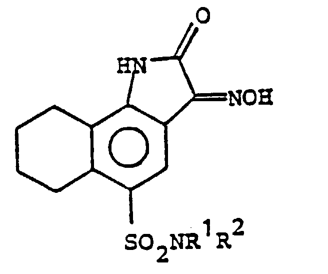 wherein
R¹ is C₁₋₆-alkyl which may be branched or cyclic;
R² is C₁₋₆-alkyl which may be branched or cyclic;
or wherein R¹ and R³ together represent -(CH₂)ₙ-, wherein n is 3, 4 or 5, comprising the step of reacting a compound having the formula 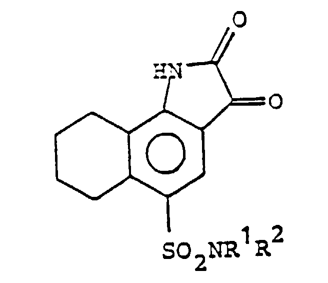 wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein n is 3, 4 or 5, with hydroxylamine, a salt or other reactive derivative thereof to form a compound of the invention.

## Claims (Claims for the following Contracting State(s): ES)

1. A method of preparing a compound having the formula (I) 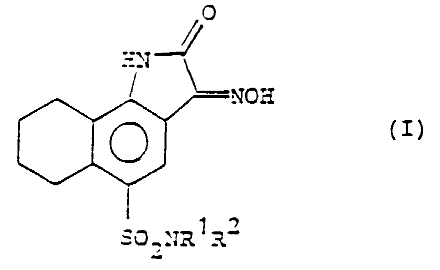 wherein
R¹ is C₁₋₆-alkyl which may be branched or cyclic;
R² is C₁₋₆-alkyl which may be branched of cyclic;
or wherein R¹ and R² together represent -(CH₂)ₙ-, wherein n is 3, 4 or 5,
comprising the step of reacting a compound having the formula 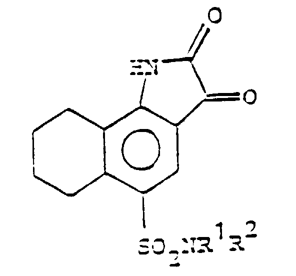 wherein
R¹ is C₁₋₆-alkyl which may be branched or cyclic;
R² is C₁₋₆-alkyl which may be branched or cyclic;
or wherein R¹ and R² together represent -(CH₂)ₙ- wherein n is 3, 4 or 5,
with hydroxylamine, a salt or other reactive derivative thereof to form a compound of the formula (I).

2. The method of claim 1 wherein
5-N,N-dimethylsulfamoyl-1H-6,7,8,9-tetrahydrobenz[g]-indole-2,3-dione-3-oxime is prepared.

3. The method of claim 1 wherein
5-(1-pyrrolidinylsulfonyl)-1H-6,7,8,9-tetrahydrobenz[g]-indole-2,3-dione-3-oxime is prepared.

4. A method of preparing a pharmaceutical composition comprising formulating a therapeutically-effective amount of a compound as prepared according to the method of claim 1 together with a pharmaceutically-acceptable carrier.

## Claims (Claims for the following Contracting State(s): GR)

1. A compound having the formula 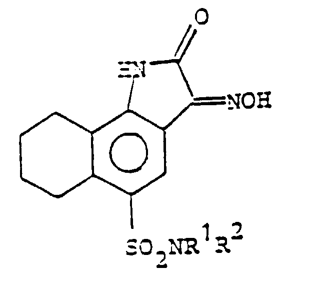 wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein n is 3, 4 or 5.

2. A compound of claim 1 which is
5-N,N-dimethylsulfamoyl-1H-6,7,8,9-tetrahydrobenz[g]-indole-2,3-dione-3-oxime.

3. A compound of claim 1 which is
5-(1-pyrrolidinylsulfonyl)-1H-6,7,8,9-tetrahydrabenz[g]-indole-2,3-dione-3-oxime.

4. A method of preparing a pharmaceutical composition comprising formulating a therapeutically-effective amount of a compound of claim 1 together with a pharmaceutically-acceptable carrier.

5. The use of a compound of claim 1, for the preparation of a medicament useful in the treatment of disorders of a mammal, including a human, responsive to the blockade of glutamic or aspartic acid receptors.

6. The use of a compound of claim 1 for the preparation of a medicament useful in the treatment of cerebrovascular disorders, Parkinsonism, anoxia, anxiety, schizophrenia, migraine, epilepsy, pain, drug addiction, Alzheimer's disease and Huntington's disease.

7. The use of claim 5 wherein the compound is 5-N,N-dimethylsulfamoyl-lH-6,7,8,9-tetrahydrobenz[g]indole-2,3-dione-3-oxime.

8. A method of preparing a compound having the formula 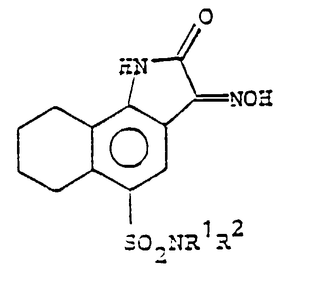 wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein n is 3, 4 or 5, comprising the step of reacting a compound having the formula 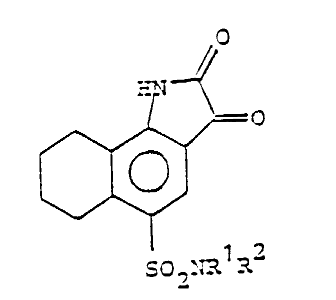
wherein
R¹ is C₁₋₆-alkyl which may be branched or cyclic;
R² is C₁₋₆-alkyl which may be branched or cyclic;
or wherein R¹ and R² together represent -(CH₂)ₙ-, wherein
n is 3, 4 or 5, with hydroxylamine, a salt or other reactive derivative thereof to form a compound of the invention.

## Patentansprüche (Patentansprüche für folgende(n) Vertragsstaat(en): AT, BE, CH, DE, DK, FR, GB, IT, LI, LU, MC, NL, PT, SE)

1. Verbindung der Formel 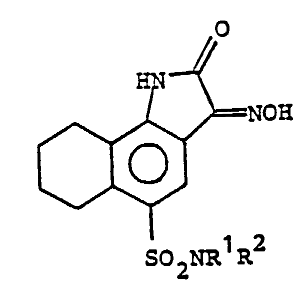 worin
R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist.

2. Verbindung nach Anspruch 1, nämlich
5-N,N-Dimethylsulfamoyl-1H-6,7,8,9-tetrahydrobenz[g]indol-2,3-dion-3-oxim

3. Verbindung nach Anspruch 1, nämlich
5-(1-Pyrrolidinylsulfonyl)-1H-6,7,8,9-tetrahydrobenz[g]indol-2,3-dion-3-oxim.

4. Pharmazeutische Zusammensetzung, die eine therapeutisch wirksame Menge einer Verbindung nach Anspruch 1 zusammen mit einem pharmazeutisch verträglichen Träger umfaßt.

5. Verwendung einer Verbindung nach Anspruch 1 zur Herstellung eines Medikaments, das bei der Behandlung von Krankheiten eines Säugers, einschließlich eines Menschen brauchbar ist, die auf die Blockierung von Glutamin- oder Asparaginsäurerezeptoren ansprechen.

6. Verwendung einer Verbindung nach Anspruch 1 zur Herstellung eines Medikaments, das bei der Behandlung von cerebrovaskulären Krankheiten, Parkinsonismus, Anoxie, Angstzuständen, Schizophrenie, Migräne, Epilepsie, Schmerzen, Drogenabhängigkeit, Alzheimerscher-Krankheit und Huntington-Krankheit brauchbar ist.

7. Verwendung nach Anspruch 5, wobei die Verbindung 5-N,N-Dimethylsulfamoyl-lH-6,7,8,9-tetrahydrobenz[g]indol-2,3-dion-3-oxim ist.

8. Verfahren zur Herstellung einer Verbindung der Formel: 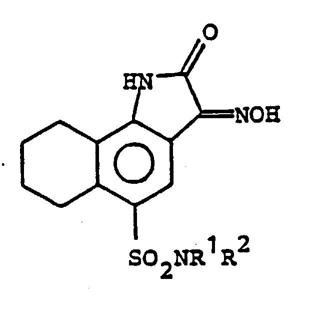
worin R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist,
das den Schritt umfaßt, eine Verbindung der Formel 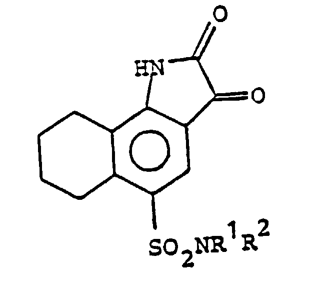
worin R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist, mit Hydroxylamin, einem Salz oder einem anderen reaktiven Derivat davon umzusetzen, wodurch eine erfindungsgemäße Verbindung hergestellt wird.

## Patentansprüche (Patentansprüche für folgende(n) Vertragsstaat(en): ES)

1. Verfahren zur Herstellung einer Verbindung der Formel (I) 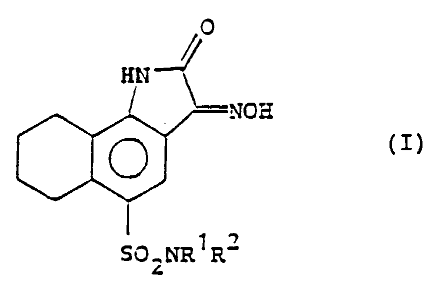 worin
R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist,
das den Schritt umfaßt, eine Verbindung der Formel 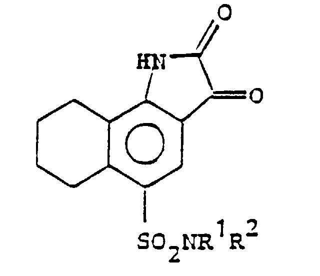
worin R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist, mit Hydroxylamin, einem Salz oder einem anderen reaktiven Derivat davon umzusetzen, wodurch eine Verbindung der Formel (I) gebildet wird.

2. Verfahren nach Anspruch 1, worin 5-N,N-Dimethylsulfamoyl-1H-6,7,8,9-tetrahydrobenz[g]indol-2,3-dion-3-oxim hergestellt wird.

3. Verfahren nach Anspruch 1, worin 5-(1-Pyrrolidinylsulfonyl)-1H-6,7,8,9-tetrahydrobenz[g]indol-2,3-dion-3-oxim hergestellt wird.

4. Verfahren zur Herstellung einer pharmazeutischen Zusammensetzung, das das Formulieren einer therapeutisch wirksamen Menge einer Verbindung, die nach dem Verfahren von Anspruch 1 hergestellt worden ist, zusammen mit einem pharmazeutisch verträglichen Träger umfaßt.

## Patentansprüche (Patentansprüche für folgende(n) Vertragsstaat(en): GR)

1. Verbindung der Formel 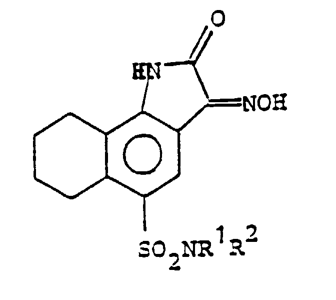 worin
R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist.

2. Verbindung nach Anspruch 1, nämlich
5-N,N-Dimethylsulfamoyl-1H-6,7,8,9-tetrahydrobenz[g]indol-2,3-dion-3-oxim

3. Verbindung nach Anspruch 1, nämlich
5-(1-Pyrrolidinylsulfonyl)-1H-6,7,8,9-tetrahydrobenz[g]-indol-2,3-dion-3-oxim.

4. Verfahren zur Herstellung einer pharmazeutischen Zusammensetzung, das das Formulieren einer therapeutisch wirksamen Menge einer Verbindung nach Anspruch 1 zusammen mit einem pharmazeutisch verträglichen Träger umfaßt.

5. Verwendung einer Verbindung nach Anspruch 1 zur Herstellung eines Medikaments, das bei der Behandlung von Krankheiten eines Säugers, einschließlich eines Menschen brauchbar ist, die auf die Blockierung von Glutamin- oder Asparaginsäurerezeptoren ansprechen.

6. Verwendung einer Verbindung nach Anspruch 1 zur Herstellung eines Medikaments, das bei der Behandlung von cerebrovaskulären Krankheiten, Parkinsonismus, Anoxie, Angstzuständen, Schizophrenie, Migräne, Epilepsie, Schmerzen, Drogenabhängigkeit, Alzheimerscher-Krankheit und Huntington-Krankheit brauchbar ist.

7. Verwendung nach Anspruch 5, wobei die Verbindung 5-N,N-Dimethylsulfamoyl-1H-6,7,8,9-tetrahydrobenz[g]indol-2,3-dion-3-oxim ist.

8. Verfahren zur Herstellung einer Verbindung der Formel: 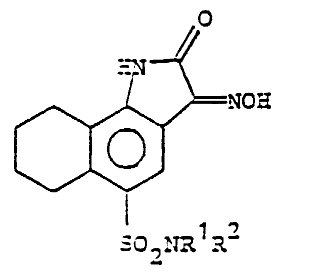 worin
R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist,
das den Schritt umfaßt, eine Verbindung der Formel 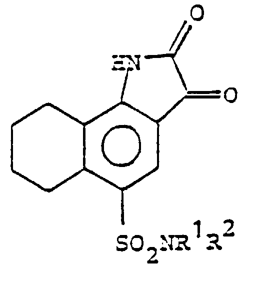
worin R¹ ein C₁₋₆-Alkylrest ist, der verzweigt oder cyclisch sein kann;
R² ein C₁₋₆-Alkylrest ist, der verzweigt oder cyclisch sein kann;
oder worin R¹ und R² zusammen -(CH₂)ₙ- darstellen, worin n 3, 4 oder 5 ist, mit Hydroxylamin, einem Salz oder einem anderen reaktiven Derivat davon umzusetzen, wodurch eine erfindungsgemäße Verbindung hergestellt wird.

## Revendications (Revendications pour l'(les) Etat(s) contractant(s) suivant(s): AT, BE, CH, DE, DK, FR, GB, IT, LI, LU, MC, NL, PT, SE)

1. Composé ayant la formule 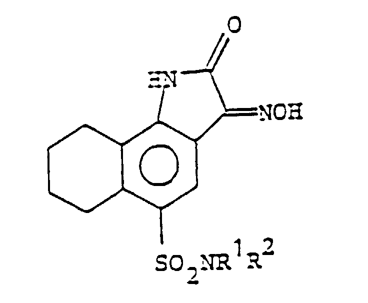 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5.

2. Composé selon la revendication 1, qui est le 5-N,N-diméthylsulfamoyl-1H-6,7,8,9-tétrahydrobenz[g]-indole-2,3-dione-3-oxime.

3. Composé selon la revendication 1, qui est le 5-(1-pyrrolidinylsulfonyl)-1H-6,7,8,9-tétrahydrobenz[g]-indole-2,3-dione-3-oxime.

4. Composition pharmaceutique comprenant une quantité thérapeutiquement efficace d'un composé selon la revendication 1 avec un excipient pharmaceutiquement acceptable.

5. Utilisation d'un composé selon la revendication 1, pour la préparation d'un médicament utile dans le traitement de troubles d'un mammifère, incluant un humain, réagissant au blocage des récepteurs de l'acide glutamique ou aspartique.

6. Utilisation d'un composé selon la revendication 1, pour la préparation d'un médicament utile dans le traitement de troubles cérébro-vasculaires, du parkinsonisme, de l'anoxie, de l'anxiété, de la schizophrénie, de la migraine, de l'épilepsie, de la douleur, de la toxicomanie, de la maladie d'Alzheimer et de la maladie de Huntington.

7. Utilisation selon la revendication 5, dans laquelle le composé est le 5-N,N-diméthylsulfamoyl-1H-6,7,8,9-tétrahydrobenz[g]indole-2,3-dione-3-oxime.

8. Procédé de préparation d'un composé ayant la formule 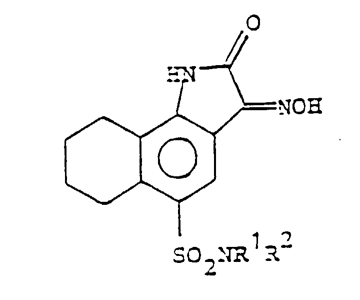 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5,
comprenant l'étape de réaction d'un composé ayant la 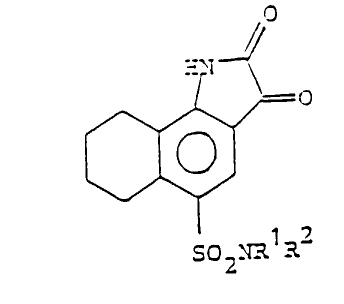
dans laquelle
R¹ est un radical alkyle en C₁ à C₆ qui peut être ramifié ou cyclique ;
R² est un radical alkyle en C1 à C6 qui peut être ramifié ou cyclique ;
ou dans laquelle R¹ et R² ensemble représentent -(CH₂)ₙ-, où n est 3, 4 ou 5,
avec de l'hydroxylamine, un sel ou autre dérivé réactif de celle-ci pour former un composé selon l'invention.

## Revendications (Revendications pour l'(les) Etat(s) contractant(s) suivant(s): ES)

1. Procédé de préparation d'un composé ayant la formule 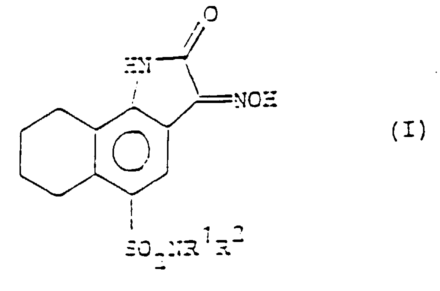 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5,
comprenant l'étape de réaction d'un composé ayant la formule 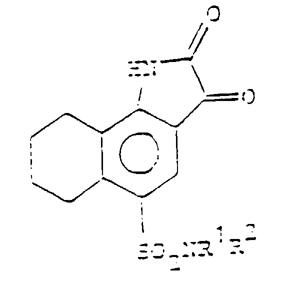 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5,
avec de l'hydroxylamine, un sel ou autre dérivé réactif de celle-ci pour former un composé de formule (I).

2. Procédé selon la revendication 1, dans lequel le 5-N,N-diméthylsulfamoyl-1H-6,7,8,9-tétrahydrobenz[g]indole-2,3-dione-3-oxime est préparé.

3. Procédé selon la revendication 1, dans lequel le 5-(1-pyrrolidinylsulfonyl)-1H-6,7,8,9-tétrahydrobenz[g]indole-2,3-dione-3-oxime est préparé.

4. Procédé de préparation d'une composition pharmaceutique, comprenant la formulation d'une quantité thérapeutiquement efficace d'un composé tel que préparé selon le procédé de la revendication 1 avec un excipient pharmaceutiquement acceptable.

## Revendications (Revendications pour l'(les) Etat(s) contractant(s) suivant(s): GR)

1. Composé ayant la formule 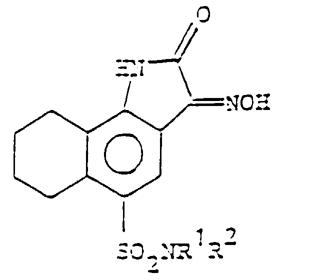 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5.

2. Composé selon la revendication 1, qui est le 5-N,N-diméthylsulfamoyl-1H-6,7,8,9-tétrahydrobenz[g]-indole-2,3-dione-3-oxime.

3. Composé selon la revendication 1, qui est le 5-(1-pyrrolidinylsulfonyl)-1H-6,7,8,9-tétrahydrobenz[g]-indole-2,3-dione-3-oxime.

4. Procédé de préparation d'une composition pharmaceutique, comprenant la formulation d'une quantité thérapeutiquement efficace d'un composé selon la revendication 1 avec un excipient pharmaceutiquement acceptable.

5. Utilisation d'un composé selon la revendication 1, pour la préparation d'un médicament utile dans le traitement de troubles d'un mammifère, incluant un humain, réagissant au blocage des récepteurs de l'acide glutamique ou aspartique.

6. Utilisation d'un composé selon la revendication 1, pour la préparation d'un médicament utile dans le traitement de troubles cérébro-vasculaires, du parkinsonisme, de l'anoxie, de l'anxiété, de la schizophrénie, de la migraine, de l'épilepsie, de la douleur, de la toxicomanie, de la maladie d'Alzheimer et de la maladie de Huntington.

7. Utilisation selon la revendication 5, dans laquelle le composé est le 5-N,N-diméthylsulfamoyl-1H-6,7,8,9-tétrahydrobenz[g]indole-2,3-dione-3-oxime.

8. Procédé de préparation d'un composé ayant la formule 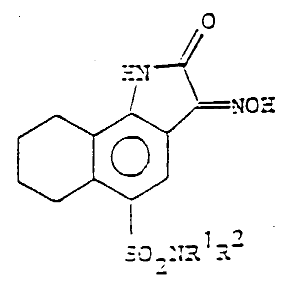 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5,
comprenant l'étape de réaction d'un composé ayant la formule 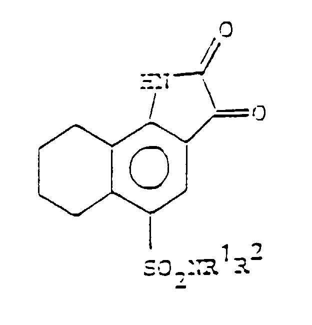 dans laquelle
R¹ est un radical alkyle en C₁ à C₆ qui peut être ramifié ou cyclique ;
R² est un radical alkyle en C₁ à C₆ qui peut être ramifié ou cyclique ;
ou dans laquelle R¹ et R² ensemble représentent -(CH₂)ₙ-, où n est 3, 4 ou 5,
avec de l'hydroxylamine, un sel ou autre dérivé réactif de celle-ci pour former un composé selon l'invention.
